(19) 

**Europäisches Patentamt**
**European Patent Office**
**Office européen des brevets**

(11) **EP 1 870 108 A1**

(12) **EUROPEAN PATENT APPLICATION**
published in accordance with Art. 153(4) EPC

(43) Date of publication:
**26.12.2007 Bulletin 2007/52**

(21) Application number: **06731286.8**

(22) Date of filing: **06.04.2006**

(51) Int Cl.:
*A61K 38/46* (2006.01)   *A61K 9/127* (2006.01)
*A61P 35/00* (2006.01)   *C12N 9/16* (2006.01)

(86) International application number:
**PCT/JP2006/307338**

(87) International publication number:
**WO 2006/109675 (19.10.2006 Gazette 2006/42)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.04.2005 JP 2005111349**

(71) Applicants:
• **Sunnyvale Company**
  **Sunnyvale CA 94085 (US)**
• **Sunaga, Susumu**
  **Kitakatsushika-gun**
  **Saitama**
  **3400214 (JP)**

(72) Inventors:
• **SHIGETA, Shiro**
  **Fukushima-shi, Fukushima 9601101 (JP)**
• **KONNO, Kenji**
  **3214337 (JP)**

(74) Representative: **Cohausz & Florack**
  **Patent- und Rechtsanwälte**
  **Bleichstrasse 14**
  **40211 Düsseldorf (DE)**

(54) **ANTITUMOR AGENT AND NOVEL DNASE**

(57)    An antitumor agent comprising as an active ingredient a DNase, and novel DNases are disclosed. The novel DNases are derived from human stomach cancer cell line MKN-28 or human cervical cancer cell line HeLa, and do not act on normal cells but specifically act on cancer cells.

EP 1 870 108 A1

**Description**

TECHNICAL FIELD

The present invention relates to an antitumor agent and a novel DNase.

BACKGROUND ART

A remarkable feature of cancer cells is an unregulated proliferation potency. A group of antitumor agents target a DNA, and it has been clarified that the damaged DNA activates a specific apoptotic pathway. Apoptosis is not a necrosis, which is a mere cell lysis, but an active cell death regulated by genes (non-patent reference 1).

As such an antitumor agent which targets a DNA and damages the DNA, for example, an alkylating agent, a topoisomerase inhibitor, and Ara-C (cytosine arabinoside) are known. The alkylating agent causes an irreversible DNA cleavage by alkylating a base portion of a DNA (adduct formation). The topoisomerase inhibitor causes a similar DNA cleavage by stabilizing an intermediate complex (cleavable complex) of a topoisomerase and a DNA. Ara-C is mistaken as deoxyadenosine (a material for a DNA) and incorporated into a DNA, to damage the DNA by inhibiting a DNA polymerase.

When a DNA is damaged by the antitumor agent, apoptosis is finally caused by a DNase, and cancer cells are excluded.

However, that a DNase per se is used as an antitumor agent, and that a DNase does not act on normal cells but specifically acts on cancer cells, is unknown. Furthermore, although a restriction enzyme is known to digest a DNA at a specific recognition site, that a restriction enzyme per se is used as an antitumor agent, and that a restriction enzyme does not act on normal cells but specifically acts on cancer cells, is unknown.

[non-patent reference 1] British Journal of Cancer, United Kingdom, 1972, Vol. 26, p. 239

DISCLOSURE OF INVENTION

PROBLEMS TO BE SOLVED BY THE INVENTION

An object of the present invention is to provide an antitumor agent which does not act on normal cells, but specifically acts on cancer cells, i.e., an antitumor agent having few adverse effects, and a novel DNase useful as an active ingredient therefor.

MEANS FOR SOLVING THE PROBLEMS

The present invention relates to an antitumor agent comprising as an active ingredient a DNase.

According to a preferred embodiment of the present invention, the DNase is used in the form of a complex of the DNase and a liposome.

According to another preferred embodiment of the present invention, the DNase is a restriction enzyme or a novel DNase, such as an MKN-28 DNase or a HeLa DNase as described below.

The present invention relates to a DNase (hereinafter referred to as "MKN-28 DNase") having the following properties:

(a) activity and substrate specificity: exhibiting an endonuclease activity;
(b) molecular weight: 48 to 43 kDa (determined by a gel filtration chromatography);
(c) optimum pH: pH 3.0 to 4.5;
(d) thermostability: the endonuclease activity is not inactivated by heating at 100°C for 10 minutes; and
(e) susceptibility to proteinase K treatment: the endonuclease activity is inactivated by a treatment with proteinase K at 37°C for 15 minutes.

The present invention relates to a DNase (hereinafter referred to as "HeLa DNase") having the following properties:

(a) activity and substrate specificity: exhibiting an endonuclease activity;
(b) molecular weight: 63 kDa (determined by a gel filtration chromatography);
(c) optimum pH: pH 3.0 to 4.5;
(d) thermostability: the endonuclease activity is inactivated by heating at 100°C for 10 minutes; and
(e) susceptibility to proteinase K treatment: the endonuclease activity is not inactivated by a treatment with proteinase K at 37°C for 15 minutes.

The present invention relates to a pharmaceutical composition comprising the MKN-28 DNase or the HeLa DNase and a pharmaceutically or veterinarily acceptable ordinary carrier or diluent.

The present invention relates to the use of a DNase in the manufacture of an antitumor agent.

The present invention relates to a method for treating or preventing cancer, comprising administering to a subject in need thereof a DNase in an amount effective in treating or preventing cancer.

EFFECTS OF THE INVENTION

The antitumor agent of the present invention exhibits an activity of inhibiting a cell proliferation with respect to various cancer cell lines, but does not exhibit the inhibitory activity to normal cells. The antitumor agent of the present invention specifically acts on cancer, and thus, has a few adverse effects.

The novel DNase according to the present invention, or a restriction enzyme which may be used as an active ingredient of the antitumor agent according to the present invention, exhibits an activity of inhibiting a cell proliferation with respect to various cancer cell lines, but does not exhibit the inhibitory activity to normal cells. Therefore, the novel DNase of the present invention or such a restriction enzyme is useful as an active ingredient of the antitumor agent according to the present invention.

BEST MODE FOR CARRYING OUT THE INVENTION

[1] Antitumor agent of the present invention

The antitumor agent of the present invention contains one or more DNases as an active ingredient. The DNase is not particularly limited, so long as it exhibits an activity of inhibiting a cell proliferation with respect to tumor cells, but does not exhibit the inhibitory activity to normal cells. As the DNase which may be used as an active ingredient of the antitumor agent according to the present invention, there may be mentioned, for example, the MKN-28 DNase of the present invention, the HeLa DNase of the present invention, DNase II, DNase I, NUC18, DNase V, DNase VI, a $Ca^{2+}/Mg^{2+}$ endonuclease (for example, human $Ca^{2+}/Mg^{2+}$ endonuclease, bovine $Ca^{2+}/Mg^{2+}$ endonuclease, or rat $Ca^{2+}/Mg^{2+}$ endonuclease), rat $Mg^{2+}$ endonuclease, rat neutral DNase, bovine nuclear endonuclease, CHO acidic endonuclease, rat DNase $\alpha$, rat DNase $\beta$, rat DNase $\gamma$, or various restriction enzymes. The DNase which may be used in the present invention includes not only an enzyme having a DNase activity alone, but also an enzyme having an enzyme activity other than the DNase activity together with the DNase activity, such as topoisomerase II (i.e., gyrase) or an integrase (for example, $\lambda$ integrase).

The DNase as used herein includes an endonuclease and an exonuclease, and an endonuclease is preferred. The antitumor agent may contain only one DNase, or a combination of two or more DNases (for example, a combination of two or more endonucleases, a combination of two or more exonucleases, or a combination of one or more endonucleases and one or more exonucleases).

Whether or not a DNase exhibits an activity of inhibiting a cell proliferation with respect to tumor cells, but does not exhibit the inhibitory activity to normal cells may be easily judged, for example, by a known method for determining an antitumor activity [for example, an MTT method (J. Virol. Methods, 20, 309-321, 1988; or Journal of Virological Methods, 20, 309, 1988)].

In the MTT method, whether or not a DNase to be judged exhibits an activity of inhibiting a cell proliferation with respect to tumor cells, but does not exhibit the inhibitory activity to normal cells may be determined, for example, by the procedures described in Example 4. More particularly, cells for evaluation, i.e., a cancer cell line (for example, an MKN-28 cell or a HeLa cell) and a normal cell (for example, an MRC-5 cell or an HEF cell) are prepared as cell suspensions. An appropriately diluted series (for example, 1/2, 1/4, 1/8, 1/16, and 1/32) of a DNase solution are poured into each well of a microplate. After each cell suspension is further added into each well, the cells are cultivated for a predetermined period (for example, 4 days). After the cultivation, an MTT [3-(4,5-dimethylthiazol-2-yl)-2,5-diphenyltetrazolium bromide; Sigma chemical Co.] solution is added into each well. After an incubation for a predetermined period (for example, at 37°C for 4 hours), a culture supernatant is removed from each well, and then an MTT formazan elution liquid [acidified isopropanol containing 10%(V/V) Triton X-100] is added into each well. After the plate is shaken, OD values are measured at wavelengths of 540 nm and 690 nm by a microplate reader, and each $IC_{50}$ value is calculated. The $IC_{50}$ value is indicated as a dilution of a test liquid or a concentration (for example, $\mu g/mL$) of a test drug which can inhibit a proliferation of a control cell (without an antitumor agent) to 50%. When the dilution is high, or when the drug concentration is low, it can be judged that the drug (substance) tested has a high activity.

As the DNase used in the present invention, the MKN-28 DNase or the HeLa DNase of the present invention, or a restriction enzyme is preferred.

It is known that a point mutation of a protooncogene located in a normal cell causes a cancer of normal cells to occur. For example, a human oncogene, activated c-ras (H-ras, K-ras, or N-ras), is a gene activated by a point mutation at a specific codon of a proto-ras gene.

For example, human lung cancer cell line A549 is a cancer cell in which the nucleotide sequence 5'-GGT-3' (Gly) of the 12th codon in c-Ki-ras2 is changed to 5'-AGT-3'(Ser): that is, the first base G among three bases in the codon is changed to A. In the A549 cell, the 11th to 12th nucleotide sequence in c-Ki-ras2 is GCTGGT before the mutation, and is GCTAGT after the mutation. The nucleotide sequence "CTAG" in the sequence GCTAGT after the mutation is a recognition sequence "C:TAG" (":" means a cleavage site) of a restriction enzyme XspI. When the antitumor agent of the present invention is used with respect to a tumor cancer caused by the same point mutation as that in the A549 cell, for example, restriction enzyme XspI or an isoschizomer thereof may be selected as the active ingredient thereof. The restriction enzyme XspI does not cleave the nucleotide sequence GCTGGT before the mutation, and thus does not act on a normal cell.

In the present invention, an appropriate restriction enzyme may be selected on the basis of a type of tumor to be treated, that is, a nucleotide sequence containing a point mutation, for example, as shown in Table 1. In Table 1, numbers in parentheses in "Protocodon before mutation" are codon numbers. In "codon after mutation", bases shown in lower case letters mean mutated bases, and recognition sites are underlined.

Table 1

| Tumor (Cell line) | Oncogene | Protocodon before mutation | Codon after mutation | Restriction enzyme |
|---|---|---|---|---|
| Lung cancer (A549) | c-Ki-ras2 | GCT GGT (11,12) | GCTaGT | XspI, BfaI FspBI, MaeI |
| Colon adenocarcinoma (HCT116) | c-Ki-ras2 | GGC GTA (13,14) | GaCGTA | MaeII HpyCH4IV |
| Pancreatic adenocarcinoma (PSN1) | c-Ki-ras2 | GGA GCT GGT (10,11,12) | GGAGCTcGT | BanII, Eco24I EcoT38I, FriOI Bsp1286I, BmyI MhlI |
| Promyelocytic leukemia (HL60) | N-ras | CAA GAA (61,62) | CtAGAA | XspI, BfaI FspBI, MaeI |
| Acute lymphatic leukemia (MOLT-4) | N-ras | GCA GGT (11,12) | GCAtGT | NlaIII, CviAII Hsp92II, FatI |
| Bladder cancer (T24) | c-Ha-ras1 | GCC GGC GGT (11,12,13) | GCCGtCGGT | Hpy99I |
| Melanoma (SK2) | c-Ha-ras1 | CAG GAG (61,62) | CtGGAG | BpmI, GsuI |
| Breast sarcoma (HS578T) | c-Ha-ras1 | GGC GGT (12,13) | GaCGGT | HpyCH4III Bst4CI, TaaI |
| Lung cancer (PR310) | c-Ki-ras2 | CAA GAG (61,62) | CAtGAG | NlaIII, CviAII Hsp92II, FatI |

The antitumor agent of the present invention may contain the DNase alone, or preferably as a complex of the DNase and a liposome.

The liposome which may be used in the present invention is, for example, a liposome prepared from phospholipids, glycolipids, or lipid molecules (such as cholesterol) and/or surfactants. A unilamellar liposome or a multilamellar liposome may be effectively used.

The phospholipids may include, for example, glycerophospholipids (for example, phosphatidylcholine, phosphatidylethanolamine, phosphatidylserine, phosphatidic acid, phosphatidylglycerol, phosphatidylinositol, or cardiolipin), or sphingophospholipids (for example, sphingomyelin, ceramide phosphorylethanolamine, or ceramide phosphorylglycerol).

The glycolipids may include, for example, glyceroglycolipids (for example, digalactosyldiglyceride or seminolipid), or sphingoglycolipids (for example, galactosylceramide or lactosylceramide).

The surfactants may include, for example, dicetyl phosphate or stearylamine.

The formulation of the antitumor agent according to the present invention containing a complex of the DNase and the liposome is not particularly limited, so long as the DNase and the liposome are contained in the antitumor agent as a complex. For example, the antitumor agent may be a mixture of the DNase and the liposome, or a formulation prepared by embedding the DNase in the liposome or encapsulating the DNase with the liposome. The embedded formulation is preferred.

The embedded formulation may be prepared, for example, in accordance with a method for embedding the DNase in the liposome. More particularly, a lipid (such as phosphatidylcholine, dicetyl phosphate, or cholesterol) is dissolved in an appropriate solvent (such as chloroform), and aliquots are poured into appropriate bottles or tubes. After the solvent is removed by blowing nitrogen gas thereinto, a DNase solution is further added and treated by a vortex mixer or the like. The whole is incubated at a predetermined temperature (for example, at 37°C) for a predetermined period (for example, for 30 minutes) to obtain a liposome containing the DNase.

When the antitumor agent of the present invention contains the complex of the DNase and the liposome (hereinafter referred to as DNase/liposome complex), a thermally-denatured immunoglobulin G (aggregated IgG) may be further contained. The thermally-denatured IgG may be prepared, for example, by dissolving 15 mg of human IgG (Human IgG Purified; Sigma Chemical Co.) in 1 mL of Ringer solution and heating the solution at 60°C for 10 minutes [Biochemistry, 15, 452, 1976]. To the DNase/liposome complex, a hundredth amount of the thermally-denatured IgG is added, and the mixture may be used, for example, at a concentration of 150μg/mL (as the final concentration of the IgG). It is known that the thermally-denatured IgG binds to an Fc receptor. Therefore, it is considered that the thermally-denatured IgG located on the surface of the liposome binds to the Fc receptor located on the membrane of a tumor cell and, as a result, the binding causes a receptor-mediated endocytosis to induce a higher activity of inhibiting cell proliferation [Biochemistry, 15(2), 452-460, 1976].

In the antitumor agent of the present invention, the DNase (preferably the DNase/liposome complex) may be administered alone or, optionally together with a pharmaceutically or veterinarily acceptable ordinary carrier or diluent, to a subject (an animal, preferably a mammal, particularly a human) in need of treatment or prevention of a cancer in an amount effective therefor. Furthermore, the DNase (preferably the DNase/liposome complex) as the active ingredient in the present invention may be used in the manufacture of an antitumor agent.

The formulation of the antitumor agent of the present invention is not particularly limited to, but may be, for example, oral medicines, such as powders, fine particles, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

The parenteral administration may be, for example, an injection such as a subcutaneous, intravenous, or intraarterial injection, or a per rectum administration. Of the parenteral formulations, an injection is preferably used. When the injections are prepared, for example, watersoluble solvents, such as physiological saline or Ringer solution, water-insoluble solvents, such as plant oil or fatty acid ester, agents for rendering isotonic, such as glucose or sodium chloride, solubilizing agents, stabilizing agents, antiseptics, suspending agents, or emulsifying agents may be optionally used, in addition to the active ingredient.

The antitumor agent of the present invention may be administered in the form of a sustained release preparation using sustained release polymers. For example, the antitumor agent of the present invention may be incorporated to a pellet made of ethylenevinyl acetate polymers, and the pellet may be surgically implanted in a tissue to be treated.

The antitumor agent of the present invention may contain the DNase (or the DNase/liposome comlex) in an amount of, but is by no means limited to, 0.01 to 99% by weight, preferably 0.1 to 80% by weight.

A dose of the antitumor agent of the present invention is not particularly limited, but may be determined dependent upon the kind of disease, the age, sex, body weight, or symptoms of the subject, a method of administration, or the like. The antitumor agent of the present invention may be orally or parenterally administered.

The antitumor agent of the present invention may be administered as a medicament or in various forms, for example, eatable or drinkable products such as health foods (preferably functional foods) or feeds. The term "foods" as used herein includes drinks.

As cancers which may be treated or prevented with the antitumor agent of the present invention, there may be mentioned, for example, stomach cancer, colon cancer, liver cancer, kidney cancer, breast cancer, oral cancer, pancreatic cancer, esophagus cancer, bladder cancer, uterine cancer, lung cancer, or leukemia.

[2] DNase of the present invention

Of the DNases which may be used as the active ingredient of the antitumor agent according to the present invention, the MKN-28 DNase and the HeLa DNase of the present invention are novel DNases.

The MKN-28 DNase of the present invention may be prepared from, for example, a human stomach cancer cell line MKN-28 (RCB1000, Riken), and has the following properties:
(a) Activity and substrate specificity: exhibiting an endonuclease activity

(b) Molecular weight: 48 to 43 kDa (determined by a gel filtration chromatography)

(c) Optimum pH: pH 3.0 to 4.5

(d) Thermostability: The endonuclease activity is not inactivated by heating at 100°C for 10 minutes.

(e) Susceptibility to proteinase K treatment: The endonuclease activity is inactivated by a treatment with proteinase K at 37°C for 15 minutes.

(f) Requirement for divalent cations: $Ca^{2+}$ or $Mg^{2+}$ is not required for the endonuclease activity. A slight dependency on $Mn^{2+}$ or $Zn^{2+}$ (0.01 to 1.0 mmol/L for $Mn^{2+}$ and 0.01 to 0.1 mmol/L for $Zn^{2+}$) is observed. $Ca^{2+}$, $Mg^{2+}$, $Mn^{2+}$, or $Zn^{2+}$ inhibits the endonuclease activity at a high concentration (10 mmol/L).

(g) Sensitivity to DNase inhibitors: Globular actin (G-actin) does not inhibit the nuclease activity. Aurintricarboxylic acid (ATA) inhibits the nuclease activity.

Citrate inhibits the nuclease activity.

Iodoacetate inhibits the nuclease activity.

Sulfate ion ($SO_4^{2-}$) inhibits the nuclease activity.

Spermine slightly inhibits the nuclease activity.

$Ca^{2+}$, $Mg^{2+}$, $Mn^{2+}$, or $Zn^{2+}$ inhibits the nuclease activity at a high concentration (10 mmol/L).

β-butyrolactone does not inhibit the nuclease activity. 1,3-butadienediepoxide does not inhibit the nuclease activity.

The MKN-28 DNase of the present invention may be prepared from the human stomach cancer cell line MKN-28 in accordance with, for example, the procedures described in Example 1 and Example 6(9). More particularly, the MKN-28 DNase of the present invention may be obtained by a process comprising the steps of:

(1) adding magnesium sulfate and ATP to an MKN-28 cell homogenate, and centrifuging the mixture to obtain a supernatant;

(2) salting out the supernatant obtained in the step (1) with 70% of ammonium sulfate, and centrifuging the whole to obtain a supernatant; and

(3) fractionating from the supernatant obtained in the step (2) a fraction having a molecular weight of 48 to 43 kDa by a gel filtration chromatography.

In the above step (3), for example, Sephacryl S-300 HR may be used to obtain the fraction of interest on the basis of the DNase activity as an index, in accordance with the procedures described in Example 2. The obtained fraction may be further purified on the basis of the DNase activity, for example, by an ion-exchange chromatography in accordance with the procedures described in Example 3.

The MKN-28 DNase of the present invention may be used as the active ingredient of the antitumor agent according to the present invention in the form of the purified DNase or a crude DNase [for example, the supernatant obtained in the step (1) or (2), or the fraction obtained in the step (3)].

The HeLa DNase of the present invention may be prepared from, for example, human cervical cancer cell line HeLa [RCB0007, Riken or ATCC CCL-2, American Type Culture Collection (ATCC)], and has the following properties:

(a) Activity and substrate specificity: exhibiting an endonuclease activity

(b) Molecular weight: 63 kDa (determined by a gel filtration chromatography)

(c) Optimum pH: pH 3.0 to 4.5

(d) Thermostability: The endonuclease activity is inactivated by heating at 100°C for 10 minutes.

(e) Susceptibility to proteinase K treatment: The endonuclease activity is not inactivated by a treatment with proteinase K at 37°C for 15 minutes.

(f) Requirement for divalent cations: $Ca^{2+}$, $Mg^{2+}$, $Mn^{2+}$, or $Zn^{2+}$ is not required for the endonuclease activity. $Ca^{2+}$ or $Mg^{2+}$ inhibits the endonuclease activity at a high concentration (10 mmol/L).

(g) Sensitivity to DNase inhibitors: G-actin does not inhibit the nuclease activity.

Aurintricarboxylic acid inhibits the nuclease activity. Citrate does not inhibit the nuclease activity. Iodoacetate inhibits the nuclease activity.

Sulfate ion ($SO_4^{2-}$) inhibits the nuclease activity.

Spermine does not slightly inhibit the nuclease activity. $Zn^{2+}$ does not inhibit the nuclease activity.

β-butyrolactone does not inhibit the nuclease activity. 1,3-butadienediepoxide does not inhibit the nuclease activity.

The HeLa DNase of the present invention may be prepared from the human cervical cancer cell line HeLa in accordance with, for example, the procedures described in Example 1 and Example 6(9). More particularly, the HeLa DNase of the present invention may be obtained by a process comprising the steps of:

(1) adding magnesium sulfate and ATP to an HeLa cell homogenate, and centrifuging the mixture to obtain a supernatant;

(2) salting out the supernatant obtained in the step (1) with 70% of ammonium sulfate, and centrifuging the whole to obtain a supernatant; and

(3) fractionating from the supernatant obtained in the step (2) a fraction having a molecular weight of 63 kDa by a gel filtration chromatography.

In the above step (3), for example, Sephacryl S-300 HR may be used to obtain the fraction of interest on the basis of the DNase activity as an index, in accordance with the procedures described in Example 2. The obtained fraction may be further purified on the basis of the DNase activity, for example, by an ion-exchange chromatography in accordance with the procedures described in Example 3.

The HeLa DNase of the present invention may be used as the active ingredient of the antitumor agent according to the present invention in the form of the purified DNase or a crude DNase [for example, the supernatant obtained in the step (1) or (2), or the fraction obtained in the step (3)].

The DNase of the present invention exhibits an activity of inhibiting a cell proliferation with respect to cancer cell lines (for example, the MKN-28 cell or the HeLa cell), but does not exhibit the inhibitory activity to normal cells (for example, human fetal lung fibroblast MRC-5 or human fetal fibroblast HEF). Therefore, the DNase of the present invention is useful as the active ingredient of the antitumor agent according to the present invention.

Various properties of the MKN-28 DNase and HeLa DNase according to the present invention and known DNase II and DNase I are shown in Tables 2 and 3.

Table 2

| Properties | MKN-28 DNase | HeLa DNase | DNase II | DNase I |
|---|---|---|---|---|
| Endonuclease activity | + | + | + | + |
| Molecular weight[a] | 48-43 kDa | 63 kDa | 37 kDa[b] 45 kDa[c] | 30 kDa |
| Optimum pH | 3.0-4.5 | 3.0-4.5 | 4.1[b] -[c] | 7.0-8.0 (Extracellular) 5.5 (ER) |
| Thermostability | Resistant (L,M buffer) | Thermolabile (A buffer) | | Inactivated (80°C, 10min) |
| Prot. K | Inactivated (M buffer) | Not (A buffer) | | |
| Divalent cations | See text | See text | None[d] None[e] | Ca, Mg, Mn (at least one) |
| DNA cleavage | | | 3'-P/5'-OH | By 10 bases 3'-OH/5'-P |

(continued)

| Properties | MKN-28 DNase | HeLa DNase | DNase II | DNase I |
|---|---|---|---|---|
| Localization | | | Lysosome | Extracellular ER |

a): Determined by a gel filtration chromatography, except for DNase I. DNase I includes four types of molecules A, B, C, and D.
b): rat liver
c): porcine spleen
d): porcine liver
e): rat spleen

Table 3

| Sensitivity to DNase inhibitors | MKN-28 DNase | HeLa DNase | DNase II | DNase I |
|---|---|---|---|---|
| G-actin | Not inhb | Not inhb | Not inhb | Inhb |
| ATA | Inhb | Inhb | | |
| Citrate | Inhb | Not inhb | | |
| Iodoacetate | Inhb | Inhb | Inhb | |
| $SO_4^{2-}$ | Inhb | Inhb | Inhb | |
| Spermine | Weakly inhb | Not inhb | | |
| Divalent cations | Inhb at 10mM (Ca,Mg,Mn,Zn) | Not inhb (Zn) | | Not inhb (Zn) |
| β-butyro-lactone | Not inhb | Not inhb | | |
| 1,3-butadiene-diepoxide | Not inhb | Not inhb | | |
| [inhb: inhibit] | | | | |

## EXAMPLES

The present invention will now be further illustrated by, but is by no means limited to, the following Examples.

Example 1: Preparation of cell extracts from various cancer cell lines

In this example, human stomach cancer cell line MKN-28 (RCB1000, Riken) and human cervical cancer cell line HeLa (RCB0007, Riken or ATCC CCL-2, ATCC) were used to prepare cell extracts, and the resulting cell extracts were further fractionated, in accordance with the following procedures. For comparison, the same procedures were repeated except that human fetal lung fibroblast MRC-5(RCB0211, Riken or ATCC CCL171, ATCC) was used as a normal cell.

More particularly, each cell line was cultivated in a Dulbecco's modified Eagle's medium, and monolayered cells after 3-day cultivation were used in the following procedures. Cells were washed with phosphate buffered saline (PBS), and collected with a cell scraper. Collected cells were suspended in PBS and centrifuged at 1200 rpm for 5 minutes. The washing treatment was repeated three times. Collected cells were suspended in 5 mL of PBS to prepare cell suspensions containing $8 \times 10^7$ cells (MKN-28 cell) and $1 \times 10^8$ cells (HeLa cell). Each cell suspension was treated with a supersonicator (Type UH-50; manufactured by MST) on ice for 5 minutes (20 kHz, 50 W) to disrupt cells. To each cell homogenate, magnesium sulfate ($MgSO_4$) and ATP were added at final concentrations of 2 mg/mL and 10 mg/mL, respectively, and allowed to stand at 37°C overnight (for 22 hours).

Each mixture was centrifuged at 3000 rpm at 4°C for 30 minutes. The resulting supernatant was salted out with 70% of ammonium sulfate $[(NH_4)_2SO_4]$ (4.72 g of ground ammonium sulfate per 10 mL of supernatant). After the salting out at 4°C for an hour, the whole was centrifuged at 1500 rpm at 4°C for 15 minutes to separate a supernatant from a pellet. The resulting supernatant and pellet dissolved in PBS were dialyzed in PBS at 4°C for 18 hours while stirring. The PBS was changed four times during the dialysis. After the dialysis, each solution was poured into cryotubes and kept at -20°C. In this connection, the above procedures were carried out as aseptically as possible. When each sample kept at -20°C was used in the following procedures, the sample was centrifuged at 10000 rpm at 4°C for 30 minutes and the resulting supernatant was used.

Hereinafter, the solution after the dialysis of the PBS solution containing the supernatant separated by the centrifugataion after the salting out is simply referred to as "the centrifugal supernatant", and the solution after the dialysis of the PBS solution containing the pellet separated by the centrifugataion after the salting out is simply referred to as "the centrifugal pellet".

Example 2: Fractionation of DNase activity (+) fraction by gel chromatography (Sephacryl S-300 HR) from centrifugal supernatant derived from cultured cells

The centrifugal supernatant prepared from the MKN-28 cells in Example 1 was fractionated under the following conditions.

Gel: Sephacryl S-300 HR (fraction range of globular proteins = $1 \times 10^4$ to $1.5 \times 10^6$; Amersham, 17-0599-01) was used. A total gel bed was 114.8 mL [= $(0.75 \text{ cm})^2 \times 3.14 \times 65 \text{ cm}$]. Column: A column of 1.5 cm (diameter) $\times$ 75 cm (height) (Econo-Column; Bio-Rad) was used.

Buffer: PBS (pH 7.2).

Flow rate: 0.4 mL/min.

Fraction: 2 mL/tube.

From collected fractions, DNase activity (+) fractions (i.e., fractions having a DNase activity) were selected, and an activity of digesting λDNA (Titer) in the selected fractions was assayed.

More particularly, an aliquot (i.e., undiluted solution) of each fraction was used to select DNase activity (+) fractions, and an activity of digesting λDNA in each positive fraction was titrated. The DNase activity and the titer were determined by an electrophoretic assay utilizing an activity of digesting λDNA as an index. Particularly, the titer was determined by diluting each fraction to four levels and analyzing an electrophoretic pattern (i.e., a degree of λDNA digestion) of λDNA digestion products.

The result is shown in Table 4. The peak of the DNase activity ranged between fractions No. 39 to No.42, particularly fraction No. 40. The peak of the activity of digesting λDNA was fraction No. 40, and the titer thereof was 80-fold. Fractions No. 39 to No.42 (four fractions) were used for the following purification step by an ion-exchange chromatography.

Table 4

| Frac. | 38 | 39 | 40 | 41 | 42 | 43 | 44 | 45 | 46 | 47 | 48 |
|---|---|---|---|---|---|---|---|---|---|---|---|
| Titer | 20 | 40(80) | 80(160) | 40 | 40 | 40 | 40 | 20 | 10 | 5 | <5 |

Example 3: Purification of MKN-28 DNase by ion-exchange chromatography

In this example, the DNase activity (+) fraction obtained in Example 2 were further purified by an ion-exchange chromatography using an Econo-Pac High Q cartridge (Stronganion, 732-0094; BIO-RAD).

Because the DNase activity (+) fraction obtained in Example 2 (i.e., a mixture of fractions No. 39 to No.42) contained PBS as a buffer, PBS was replaced with a buffer for High Q by dialysis, and the resulting liquid was filtered through a membrane filter (0.45 $\mu$m). After confirming that the filtrate exhibited the DNase activity, the filtrate was applied to the Econo-Pac High Q cartridge. Elution was carried out by a concentration gradient of NaCl, that is, 50mmol/L-Tris-HCl (pH7.5) containing 0.02 to 0.3 mol/L NaCl was used as a buffer for elution. The flow rate was 1.5 mL/2 min./fraction.

The DNase activity in each fraction was determined by an electrophoretic assay utilizing an activity of digesting λDNA as an index. As samples for the assay, an aliquot (i.e., undiluted solution) of each fraction was used. After the activity of digesting λDNA in each fraction was determined, the titer of DNase activity (+) fractions was determined to detect a peak of the DNase activity.

The result is shown in Table 5. The peak of the DNase activity ranged from fraction No. 5 (NaCl concentration = 0.08 mol/L) to fraction No.6 (NaCl concentration = 0.10 mol/L).

.

Table 5

| Frac. | 1 | 2 | 3 | 4 | 5 | 6 | 7 | 8 | 9 | 10 | 11 |
|---|---|---|---|---|---|---|---|---|---|---|---|
| NaCl (mol/L) | 0.01 | 0.02 | 0.04 | 0.06 | 0.08 | 0.10 | 0.12 | 0.14 | 0.16 | 0.18 | 0.20 |
| Digestion of λDNA | - | ± | + | ++ | +++ | +++ | +++' | - | ± | - | - |

Example 4: Analysis of cell proliferation inhibitory effect of fractions derived various cancer cell lines

In this example, an activity of inhibiting a cell proliferation in the centrifugal supernatants and the centrifugal pellets derived from various cancer cell lines and normal cells prepared in Example 1 was evaluated by an MTT method. As cells for evaluation, the MKN-28 cell and the HeLa cell were used as cancer cell lines, and the MRC-5 cell was used as a normal cell. After each cell line was cultured for 3 days, cells were treated with trypsin, and dispersed cells were washed with PBS by centrifugation. Washed cells were suspended in a Dulbecco's modified Eagle's MEM medium containing 8% fetal calf serum (hereinafter referred to as GM), and counted with a hemocytometer. Each cell suspension was adjusted with the GM to cell densities of 5000 cells/50 $\mu$L and 2500 cells/50 $\mu$L (cancer cell lines) or 10000 cells/ 50 $\mu$L and 5000 cells/50 $\mu$L (MRC-5 cell).

To each well in a 96-well flat-bottomed plate, diluted series (undiluted, 1/2, 1/4, 1/8, 1/16, and 1/32; 50 $\mu$L/well) of each centrifugal supernatant or centrifugal pellet prepared in Example 1 were added. Further, each cell suspension was added to each well, and the plate was incubated at 37°C in a $CO_2$ incubator. After 3 or 4 days from the beginning of the incubation, 20 $\mu$L/well of an MTT solution was added to each well, and the plate was incubated at 37°C for 3 hours. The culture supernatant was removed from each well, and 100 $\mu$L of an MTT formazan elution liquid was added to each well. After the plate was shaken at room temperature for 5 minutes, OD values were measured at wavelengths of 540 nm and 690 nm by a microplate reader, and each $IC_{50}$ value was calculated.

As a control, PBS was used instead of the diluted series of each centrifugal supernatant or centrifugal pellet. For comparison, 5-fold-diluted series (100 $\mu$g/mL, 20 $\mu$g/mL, 4 $\mu$g/mL, 0.8 $\mu$g/mL, 0.16 $\mu$g/mL, and 0.032 $\mu$g/mL) of 5-fluorouracil (5-FU) were used instead of the diluted series of each centrifugal supernatant or centrifugal pellet. The $IC_{50}$ values when the cancer cell line MKN-28 was used as the cell for evaluation and the centrifugal supernatant and the centrifugal pellet derived from the MKN-28 cell were used as the cell fraction are shown in Table 6. Furthermore, the results of the control (PBS) and the comparison (5-FU) are shown in Table 6. Each $IC_{50}$ value shown in Table 6 (or Tables 7 to 10 described below) is an average of plural (two to four) values. Each $IC_{50}$ value is shown as a dilution (i.e., relative value) of a test liquid [or a concentration of a test drug ($\mu$g/mL for 5-FU)] which can inhibit a proliferation of a control cell (without an antitumor agent) to 50%.

As apparent from Table 6, the centrifugal supernatant and the centrifugal pellet derived from the MKN-28 cell exhibited an activity of inhibiting a cell proliferation with respect to the MKN-28 cell.

Table 6

| Cell for evaluation | MKN (3 days) | | MKN (4 days) | |
|---|---|---|---|---|
| Cells/well | 5000 | 2500 | 5000 | 2500 |
| MKN sup. | 2.8 | <2 | 11.7 | 5.33 |
| MKN pellet | 4.18 | 2.48 | 10.1 | 6.9 |
| PBS | 2.2 | <2 | 2.3 | 3.0 |
| 5-FU | 5.9 | 4.6 | 14.8 | 3.7 |

The $IC_{50}$ values when the cancer cell line HeLa was used as the cell for evaluation and the centrifugal supernatant and the centrifugal pellet derived from the HeLa cell were used as the cell fraction are shown in Table 7. As apparent from Table 7, the centrifugal supernatant and the centrifugal pellet derived from the HeLa cell exhibited an activity of inhibiting a cell proliferation with respect to the HeLa cell.

Table 7

| Cell for evaluation | HeLa (3 days) | | HeLa (4 days) | |
|---|---|---|---|---|
| Cells/well | 5000 | 2500 | 5000 | 2500 |
| HeLa sup. | 4.8 | 2.4 | 3.2 | 3.8 |
| HeLa pellet | 11.1 | 6.7 | 10.2 | 21.5 |
| PBS | <2 | <2 | <2 | <2 |
| 5-FU | 7.3 | 5.0 | 3.6 | 3.6 |

The $IC_{50}$ values when the normal cell MRC-5 was used as the cell for evaluation and the centrifugal supernatant and the centrifugal pellet derived from the MRC-5 cell were used as the cell fraction are shown in Table 8. As apparent from Table 8, the centrifugal supernatant and the centrifugal pellet derived from the MRC-5 cell did not exhibit an activity of inhibiting a cell proliferation with respect to the MRC-5 cell.

Table 8

| Cell for evaluation | MRC (3 days) | | MRC (4 days) | |
|---|---|---|---|---|
| Cells/well | 10000 | 5000 | 10000 | 5000 |
| MRC sup. | <2 | <2 | <2 | <2 |
| MRC pellet | 2.8 | 2.5 | 2.8 | 2.7 |
| PBS | 2.2 | 2.2 | 2.2 | 2.3 |
| 5-FU | >100 | >100 | >100 | 92.0 |

The $IC_{50}$ values when three types of cells were used as the cell for evaluation and three types of centrifugal supernatants derived from each cell were used as the cell fraction are shown in Table 9 (3-day cultivation) and Table 10 (4-day cultivation).

As apparent from Tables 9 and 10, each centrifugal supernatant exhibited an activity of inhibiting a cell proliferation with respect to the cancer cell lines MKN-28 and HeLa, but did not exhibit the activity with respect to the normal cell MRC-5. The sensitivity to the centrifugal supernatants was as follows:

```
MKN-28 cell > HeLa cell > MRC-5 cell.
```

As shown in Example 6 described below, the centrifugal supernatants derived from the MKN-28 cell and the HeLa cell digested DNA, and thus were nucleases.

Table 9

| [3-day cultivation] Cell for evaluation | MKN | | HeLa | | MRC | |
|---|---|---|---|---|---|---|
| Cells/well | 5000 | 2500 | 5000 | 2500 | 10000 | 5000 |
| MKN sup. | 2.6 | 2.5 | 2.9 | 3.4 | <2 | <2 |
| HeLa sup. | 2.6 | 2.2 | 4.8 | 2.9 | <2 | <2 |
| MRC sup. | 2.7 | 2.2 | 3.6 | 3.5 | <2 | 2.1 |
| PBS | <2 | <2 | <2 | 2.1 | <2 | <2 |
| 5-FU | 6.0 | 4.6 | 12.0 | 7.0 | >100 | >100 |

Table 10

| [4-day cultivation] Cell for evaluation | MKN | | HeLa | | MRC | |
|---|---|---|---|---|---|---|
| Cells/well | 5000 | 2500 | 5000 | 2500 | 10000 | 5000 |
| MKN sup. | 11.7 | 6.3 | 2.3 | 3.3 | <2 | <2 |
| HeLa sup. | 5.8 | 5.0 | 2.6 | 3.4 | <2 | <2 |
| MRC sup. | 5.0 | 4.1 | 2.5 | 2.7 | <2 | <2 |
| PBS | 2.2 | 2.4 | <2 | <2 | <2 | <2 |
| 5-FU | 11.6 | 4.7 | 13.0 | 6.2 | >100 | 92.0 |

<u>Example 5: Effects of MKN- and HeLa-centrifugal supernatants treated with DNase, RNase, and heating on MKN-28 cell proliferation</u>

In this example, after the centrifugal supernatants derived from the cancer cell lines MKN-28 and HeLa, prepared in Example 1, were heated or treated with an RNase or a DNase, an activity thereof for inhibiting a cell proliferation with respect to the cancer cell line MKN-28 was examined to clarify the properties of each centrifugal supernatant. The activity of inhibiting a cell proliferation was measured in accordance with the method described in Example 4. The result ($IC_{50}$ values) is shown in Table 11. The symbol "-" in Table 11 means that the measurement was not carried out.

The RNase treatment ("RN" in Table 11) was carried out by treating 100 $\mu$L of each cell extract with 10 $\mu$g of RNase (R 5125, Type III A; SIGMA) at 37°C for 1 hour.
The DNase treatment ("DN" in Table 11) was carried out by treating 100 $\mu$L of each cell extract with 134 units of DNase (Lot 18600k; Nippon Gene) at 37°C for 1 hour.

As a control for the RNase treatment and the DNase treatment, each cell extract was incubated at 37°C for 1 hour ("37°C" in Table 11).
To check the cell-proliferation inhibitory activity of the RNase or the DNase, a treatment with the RNase and PBS ("RN-Cont" in Table 11) and a treatment with the DNase and PBS ("DN-Cont" in Table 11) were carried out.

As the heating treatment, each cell extract was heated at 56°C for 30 minutes ("56°C" in Table 11).
As a control, each cell extract was treated with PBS ("PBS" in Table 11).

As shown in Table 11, the cell-proliferation inhibitory activity of each centrifugal supernatant derived cancer cell lines was not inactivated with the RNase or DNase treatment or by the heating treatment at 56°C for 30 minutes. Furthermore, the activity was not inactivated with an RNase H treatment (data not shown). From the results, it was found that a factor showing the cell-proliferation inhibitory activity in each centrifugal supernatant derived cancer cell lines was (1) not a nucleic acid, and (2) not inactivated with the heating treatment. In this connection, the results depended on a reaction buffer, as shown in Example 6 described below.

<div align="center">

Table 11

$IC_{50}$ value (fold)

| | RN | DN | 37°C | NT | RN-Cont | DN-Cont | 56°C | PBS |
|---|---|---|---|---|---|---|---|---|
| MKN sup. | 7.6 | 7.6 | 7.9 | 7.8 | 2.5 | 2.3 | 8.0 | 2.4 |
| HeLa sup. | 7.9 | 8.0 | 7.9 | 8.3 | - | - | 9.0 | - |
| [NT: not treated] | | | | | | | | |

</div>

<u>Example 6: Analysis of properties of centrifugal supernatants derived from cancer cell lines</u>

(1) Nuclease activity against λDNA and genomic DNA of MKN-28 cell

In this example, an activity of digesting DNA in each centrifugal supernatant derived from the cancer cell lines MKN-28 and HeLa prepared in Example 1 was examined.
Because each centrifugal supernatant contained PBS, PBS was replaced with a TE buffer (10 mmol/L Tris-HCl, 1 mmol/L EDTA, pH8.0). As DNAs to be digested, λDNA (48502bp; Takara) and a genomic DNA from the MKN-28 cell were used. The genomic DNA was extracted from the MKN-28 cell with a commercially available kit (Genomic Prep. TM cells and Tissue DNA Isolation Kit; Amersham pharmacia biotech.).

As reaction buffers, an L (Low) buffer (without NaCl), an M (Medium) buffer (50 mmol/L NaCl), and an H (High) buffer (100 mmol/L NaCl) were used. The reaction buffers were prepared by adding the above-mentioned amount of NaCl to a basic composition [10 mmol/L Tris-HCl (pH7.5), 10 mmol/L $MgCl_2$, and 1 mmol/L DTT (Dithiothreitol)].
Each reaction was carried out at 37°C for 1 hour, and a degree of DNA digestion was observed by electrophoresis.

The centrifugal supernatant derived from the MKN-28 cell digested both DNAs (i.e., λDNA and the genomic DNA derived from the MKN-28 cell) in the L or M buffer, but did not digest both DNAs in the H buffer.
The centrifugal supernatant derived from the HeLa cell did not digest both DNAs (i.e., λDNA and the genomic DNA derived from the MKN-28 cell) in the L, M, or H buffer.

(2) Optimum buffer

The procedure described in Example 6(1) was repeated except that an A buffer [50 mmol/L Mes buffer (pH 5.8), 1 mmol/L $CaCl_2$, and 3 mmol/L $MgCl_2$] and a B buffer [50 mmol/L Mops buffer (pH 7.0), 1 mmol/L $CaCl_2$, and 3

mmol/L MgCl$_2$] were used as the reaction buffer.

The centrifugal supernatant derived from the HeLa cell digested both DNAs (i.e., λDNA and the genomic DNA derived from the MKN-28 cell) in the A buffer, but did not digest both DNAs in the B buffer.

The nuclease activity in the centrifugal supernatant derived from the MKN-28 cell was remarkable in the A buffer, and slightly digested λDNA in the B buffer.

(3) Resistance to heating treatment

Each centrifugal supernatant was heated at 100°C for 10 minutes, and the nuclease activity was examined. After the heating treatment, the centrifugal supernatant derived from the MKN-28 cell digested both DNAs (i.e., λDNA and the genomic DNA derived from the MKN-28 cell) in the L or M buffer. That is, the centrifugal supernatant derived from the MKN-28 cell was not inactivated by the heating treatment.

The centrifugal supernatant derived from the HeLa cell digested λDNA in the A buffer before the heating treatment, but did not digest λDNA after the heating treatment. That is, the centrifugal supernatant derived from the HeLa cell was inactivated by the heating treatment.

(4) Resistance to proteinase K treatment

Each centrifugal supernatant was treated with proteinase K at 37°C for 15 minutes, and the nuclease activity was examined.

The centrifugal supernatant derived from the MKN-28 cell digested λDNA in the M buffer before the treatment with proteinase K, but did not digest λDNA after the treatment with proteinase K. That is, the centrifugal supernatant derived from the MKN-28 cell was inactivated by the treatment with proteinase K.

The centrifugal supernatant derived from the HeLa cell digested λDNA in the A buffer after the treatment with proteinase K. That is, the centrifugal supernatant derived from the HeLa cell was not inactivated by the treatment with proteinase K. The results described in Example 6(1) to 6(4) are shown in Table 12.

Table 12

| Sup. | λDNA | MKN-28 DNA | Heating | Proteinase K | Optimum buffer |
|------|------|-----------|---------|--------------|----------------|
| MKN | D | D | NI (heat-resistant) | IA | A buffer (L,M buffer) |
| HeLa | D | D | IA (thermolabile) | NI | A buffer |
| [D: Digested, NI: Not inactivated, IA: Inactivated] | | | | | |

(5) Optimum pH

The optimum pH of the nuclease activity in each centrifugal supernatant was examined on the basis of the activity of digesting λDNA as an index.

λDNA was digested in 50 mmol/L buffers [Acetate-HCl (pH 1.0-5.5), MOPS-NaOH (pH 6.0-7.0), and Tris-HCl (pH 7.5-8.5); in increment of pH 0.5 from pH 1.0 to pH 8.5] supplemented with 1 mmol/L DTT at 37°C for 30 minutes, and the digestion was observed by electrophoresis.

Both centrifugal supernatants (i.e., the centrifugal supernatant derived from the MKN-28 cell and the centrifugal supernatant derived from the HeLa cell) almost completely digested λDNA at pH 4.5 or less. When the same procedure was repeated except that no centrifugal supernatants were added, λDNA was digested at pH 2.5 or less. From the results, the optimum pH of each centrifugal supernatant was pH 3.0 to 4.5.

(6) Requirement for divalent cations

The requirement for divalent cations (Ca$^{2+}$, Mg$^{2+}$, Mn$^{2+}$, and Zn$^{2+}$) in each centrifugal supernatant was examined on the basis of the activity of digesting λDNA as an index.

After each centrifugal supernatant was dialyzed in PBS (Ca$^{2+}$ and Mg$^{2+}$ free), the titer thereof was measured to determine the optimum dilution concentration. As a result, the optimum dilution concentration of the centrifugal supernatant derived from the MKN-28 cell and the centrifugal supernatant derived from the HeLa cell were 1/5 and 1/3, respectively.

An acetate-HCl buffer (Ca$^{2+}$ and Mg$^{2+}$ free) supplemented with 3 mmol/L MgCl$_2$ was used for examining the requirement for Ca$^{2+}$. An acetate-HCl buffer (Ca$^{2+}$ and Mg$^{2+}$ free) supplemented with 3 mmol/L CaCl$_2$ was used for examining the requirement for Mg$^{2+}$. An acetate-HCl buffer (Ca$^{2+}$ and Mg$^{2+}$ free) supplemented was used for examining the requirement

for $Mn^{2+}$ or $Zn^{2+}$. The pH of each buffer for the centrifugal supernatant derived from the MKN-28 cell was pH 4.5. The pH of each buffer for the centrifugal supernatant derived from the HeLa cell was pH 4.0.

The result in the centrifugal supernatant derived from the MKN-28 cell is shown in Table 13. The result in the centrifugal supernatant derived from the HeLa cell is shown in Table 14. In Table 13, the number of "+" indicates a degree of λDNA digestion, and "(-)" means that the assay was not performed. In addition, the results in the absence of $Ca^{2+}$ and $Mg^{2+}$ and the results in the absence of centrifugal supernatants (i.e., only buffer) were "+++" and "-" (not digested), respectively (data not shown).

As shown in Table 13, the centrifugal supernatant (1/5 dilution) derived from the MKN-28 cell required no divalent cations ($Ca^{2+}$, $Mg^{2+}$, $Mn^{2+}$, or $Zn^{2+}$), and $Ca^{2+}$, $Mg^{2+}$, or $Zn^{2+}$ inhibited the activity at a high concentration (10 mmol/L or more).

In addition, the same procedure was repeated, except that the centrifugal supernatant derived from the MKN-28 cell was diluted to 1/40 (i.e., limited concentration for detecting the λDNA digestion), to confirm the requirement for divalent cations at a high sensitivity. $Ca^{2+}$ or $Mg^{2+}$ was not required. A slight dependency on $Mn^{2+}$ (0.01 to 1.0 mmol/L) or $Zn^{2+}$ (0.01 to 0.1 mmol/L) was observed. $Ca^{2+}$, $Mg^{2+}$, $Mn^{2+}$, or $Zn^{2+}$ inhibited the activity at a high concentration (10 mmol/L or more).

As shown in Table 14, the centrifugal supernatant (1/3 dilution) derived from the HeLa cell required no divalent cations ($Ca^{2+}$, $Mg^{2+}$, $Mn^{2+}$, or $Zn^{2+}$), and $Ca^{2+}$ or $Mg^{2+}$ inhibited the activity at a high concentration (10 mmol/L or more). When the same procedure was repeated, except that the centrifugal supernatant derived from the HeLa cell was diluted to 1/20 (i.e., limited concentration for detecting the λDNA digestion), $Ca^{2+}$, $Mg^{2+}$, $Mn^{2+}$, or $Zn^{2+}$ was not required, and $Ca^{2+}$, $Mg^{2+}$, $Mn^{2+}$, or $Zn^{2+}$ inhibited the activity at a high concentration (10 mmol/L or more).

Table 13

| Concentration of divalent cations (mmol/L) | 0 | 0.01 | 0.03 | 0.1 | 0.3 | 1.0 | 3.0 | 10 | 30 |
|---|---|---|---|---|---|---|---|---|---|
| $Ca^{2+}$ | +++ | (-) | +++ | (-) | +++ | (-) | +++ | + | ± |
| $Mg^{2+}$ | +++ | (-) | +++ | (-) | +++ | (-) | +++ | + | ± |
| $Mn^{2+}$ | +++ | +++ | (-) | +++ | (-) | +++ | (-) | +++ | (-) |
| $Zn^{2+}$ | +++ | +++ | (-) | +++ | (-) | +++ | (-) | + | (-) |

Table 14

| Concentration of divalent cations (mmol/L) | 0 | 0.01 | 0.03 | 0.1 1 | 0.3 | 1.0 | 3.0 0 | 10 | 30 |
|---|---|---|---|---|---|---|---|---|---|
| $Ca^{2+}$ | +++ | (-) | +++ | (-) | +++ | (-) | +++ | ++ | ± |
| $Mg^{2+}$ | +++ | (-) | +++ | (-) | +++ | (-) | +++ | ++ | ± |
| $Mn^{2+}$ | +++ | +++ | (-) | +++ | (-) | +++ | (-) | +++ | (-) |
| $Zn^{2+}$ | +++ | +++ | (-) | +++ | (-) | +++ | (-) | +++ | (-) |

(7) Sensitivity to DNase inhibitors

The sensitivity of each centrifugal supernatant to various inhibitors was examined on the basis of the activity of digesting λDNA as an index.

After the centrifugal supernatant derived from the MKN-28 cell was diluted to 1/5 to prepare a sample, the sample was incubated in the presence of each inhibitor at pH 4.5 at 37°C for 30 minutes, and the λDNA digestion was tested by electrophoresis. The centrifugal supernatant derived from the HeLa cell was diluted to 1/3 to prepare a sample, the sample was incubated in the presence of each inhibitor at pH 4.0 at 37°C for 30 minutes, and the λDNA digestion was tested by electrophoresis.

Effects of each inhibitor for λDNA digestion are shown in Table 15 and Table 16.

In Table 15, "(G)" means G-actin (Globular actin, derived from bovine muscle; Sigma), and the numbers "1,10,100" shown under "(G)" mean concentrations of G-actin ($\mu$g/mL). "(A)" means ATA (Aurintricarboxylic acid; Wako), and the numbers "1,10,100" shown under "(A)" mean concentrations of ATA ($\mu$mol/L). "(C)" means citrate (sodium citrate; Wako), "(I)" means Iodoacetate (Nakarai), "(SO)" means $SO_4^{2-}$ ($MgSO_4$; Wako), "(Zn)" means $Zn^{2+}$ ($ZnCl_2$; Wako), and "(S)" means spermine. "(B)" means β-butyrolactone (Tokyo Kasei Kogyo), and the numbers "0.1,1.0,10" shown under "(B)" mean concentrations of β-butyrolactone (mmol/L). "(BD)" means 1,3-butadienediepoxide (Tokyo Kasei Kogyo), and the numbers "0.1,1.0,10" shown under "(B)" mean concentrations of 1,3-butadienediepoxide (mmol/L).

The number of "+" indicates a degree of the activity for inhibiting the λDNA digestion. "-" means no inhibitory activity of the λDNA digestion, and "±" means a slight inhibitory activity of the λDNA digestion.

Table 15

| | (G) | | | (A) | | | (C) | (I) | (SO) | (Zn) | (S) |
|---|---|---|---|---|---|---|---|---|---|---|---|
| | 1 | 10 | 100 | 1 | 10 | 100 | | | | | |
| MKN | - | - | - | ± | ++ | +++ | ++ | +++ | ++ | ++ | + |
| HeLa | - | - | - | - | - | ++ | - | ++ | + | ± | - |

Table 16

| | (B) | | | (BD) | | |
|---|---|---|---|---|---|---|
| | 0.1 | 1.0 | 10 | 0.1 | 1.0 | 10 |
| MKN | - | - | - | - | - | - |
| HeLa | - | - | - | - | - | - |

(8) Digestion of circular double-stranded DNA

To determine which the nuclease activity of each centrifugal supernatant was an endonuclease activity or an exonuclease activity, a circular double-stranded DNA (plasmid pACYC184; Nippon Gene, 13-0220) was treated with each centrifugal supernatant to examine the digestion as an index.

The centrifugal supernatant derived from the MKN-28 cell or the HeLa cell digested the plasmid pACYC184. The results showed that both centrifugal supernatants were endonucleases.

(9) Molecular weight (determined by a gel filtration chromatography)

Each centrifugal supernatant was fractionated by a gel filtration chromatography to purify each DNase of the present invention and determine the molecular weight thereof. The conditions were as follows:

Column: A column of 1.5 cm (diameter) × 75 cm (height) (Econo-Column; Bio-Rad) was used.
Gel: Sephacryl S-300 HR (fraction range of globular proteins = $1 \times 10^4$ to $1.5 \times 10^6$; Amersham) was used. A total gel bed was 114.8 mL [= $(0.75 \text{ cm})^2 \times 3.14 \times 65 \text{ cm}$].
Buffer: PBS (pH 7.2).
Flow rate: 0.4 mL/min.
Fraction: 2 mL/tube.
Calculation of molecular weight: A commercially available kit (Gel Filtration Calibration Kits; Amersham Bioscience) was used. As molecular makers, albumin (M.W. = 67000), ovalbumin (M.W. = 43000), and chymotrypsinogen (M.W. = 25000) were used.

After the fractionation, the titer of each fraction was measured on the basis of the activity of digesting λDNA as an index to determine the peak fraction of each DNase of the present invention.

When the centrifugal supernatant derived from the MKN-28 cell was used, the peak fraction was fractions No. 25 and No. 26 and the molecular weight thereof was 48 to 43 kDa.

When the centrifugal supernatant derived from the HeLa cell was used, the peak fraction was fraction No. 25 and the molecular weight thereof was 63 kDa.

Example 7: Evaluation of liposome formulation containing centrifugal supernatant derived from MKN-28 cell: activity of inhibiting MKN-28 cell proliferation, cytotoxicity of liposome, reaction buffer, and pH of reaction buffer

The centrifugal supernatant derived from the MKN-28 cell was a DNase, as shown in Example 6. The optimum pH of the centrifugal supernatant derived from the MKN-28 cell (MKN-28 DNase) was acidic (pH 3.0 to 4.5), as previously determined by electrophoresis on the basis of the λDNA digestion as an index. Various acidic buffers having a pH acceptable to cell proliferation were used to prepare liposome formulations containing the MKN-28 DNase, and the activity thereof for inhibiting cell proliferation was examined.

The liposome was prepared by dissolving phosphatidylcholine (L-α-phosphatidylcholine, derived from egg yolk; Nakarai), dicetyl phosphate (Sigma), and cholesterol (ICN Biochemical Inc.) [7:2:1 (mole ratio)] in chloroform and evaporating chloroform.

More particularly, 70 μmol of phosphatidylcholine, 20 μmol of dicetyl phosphate, and 10 μmol of cholesterol was dissolved in 1 mL of chloroform (7:2:1; 100 μmol/mL). After the mixture was diluted with chloroform to 1/16 (6.25 μmol/mL), 50 μL of aliquots were added to sample bottles (0.3125 μmol/bottle) . Nitrogen gas was blown into each sample bottle while rotating, to evaporate the chloroform, which was further evaporated under a reduced pressure to prepare a thin layer of liposome. To each sample bottle, 500 μL of a reaction buffer containing the DNase (or only a buffer) was added (0.3125 μmol/500 μL = 0.625 μmol/mL).

As the reaction buffer contained together with the DNase in the liposome, various PBSs were used in view of isotonicity and composition. The PBS (pH 6.0) and the PBS (pH 7.2) may be easily prepared by changing a ratio of $KH_2PO_4$ and $Na_2HP0_4$ contained in PBS. Whether or not PBSs are appropriate for the reaction buffer was examined. Furthermore, whether or not the difference in pH of PBSs affects the activity of inhibiting cell proliferation was examined.

A membrane filter for concentration (Amicon) was used to concentrate the centrifugal supernatant derived from the MKN-28 cell, and the buffer was replaced with the PBS (pH 6.0) or the PBS (pH 7.2). To each sample bottle containing the thin layer of liposome, 500 μL of the PBS (pH 6.0 or 7.2) or the MKN-28-DNase-containing PBS (pH 6.0 or 7.2) was added and vortexed. A thermally-denatured human IgG (final concentration = 150 μg/mL) was further added, and the whole was incubated at 37°C for 30 minutes to prepare various thermally-denatured IgG coated (hereinafter sometimes referred to as "Agg-IgG coated") liposome formulations containing the MKN-28 DNase (pH 6.0 or 7.2). The thermally-denatured human IgG was prepared by dissolving 15 mg of human IgG (Human IgG Purified; Sigma Chemical Co.) in 1 mL of Ringer solution and heating at 60°C for 10 minutes.

The MKN-28 cell and a normal cell [human fetal fibroblast HEF (J. Infect. Dis., 163, 270-275, 1991)] were used to evaluate the cell-proliferation inhibitory activity of each Agg-IgG coated liposome formulation containing the MKN-28 DNase (pH 6.0 or 7.2) by the MTT method.

To each well in a 96-well microplate, double-diluted series (undiluted, 1/2, 1/4, 1/8, 1/16, and 1/32; 50 μL/well) of each Agg-IgG coated liposome formulation containing the MKN-28 DNase prepared with the GM were added. Further, 50 μL of a suspension of the MKN-28 cell or the HEF cell (5000 cells/50 μL) was added, and incubated at 37°C in a $CO_2$ incubator without changing the culture medium. After 4 days from the beginning of the incubation, each $IC_{50}$ value was calculated by the MTT method.

As a control, double-diluted series of Agg-IgG coated liposome (without the MKN-28 DNase), double-diluted series of MKN-28 DNase (without the Agg-IgG coated liposome), and buffers [PBS (pH 6.0) and PBS (pH 7.2)] were used, instead of the double-diluted series of Agg-IgG coated liposome formulation containing the MKN-28 DNase.

The results are shown in Table 17 and Table 18. In Tables 17 and 18, "LP-DN", "LP(-DN)", "DN(-LP)", and "PBS" mean the Agg-IgG coated liposome formulation containing the MKN-28 DNase, the Agg-IgG coated liposome (without the MKN-28 DNase), the MKN-28 DNase (without the Agg-IgG coated liposome), and only PBS (buffer), respectively. Each $IC_{50}$ value shown in Tables 17 and 18 is an average of plural (two to four) values. The activities of inhibiting a proliferation of the cancer cell line MKN-28 and human fetal fibroblast HEF as a normal cell are shown in Table 17 and Table 18, respectively.

Table 17

| [MKN-28] | LP-DN | | LP(-DN) | | DN(-LP) | | PBS | |
|---|---|---|---|---|---|---|---|---|
| pH | 6.0 | 7.2 | 6.0 | 7.2 | 6.0 | 7.2 | 6.0 | 7.2 |
| $IC_{50}$ | 24.5 | 26.0 | 4.3 | 3.5 | 15.0 | 15.00 | 2.7 | 2.5 |

Table 18

| [HEF] | LP-DN | | LP (-DN) | | DN(-LP) | | PBS | |
|---|---|---|---|---|---|---|---|---|
| pH | 6.0 | 7.2 | 6.0 | 7.2 | 6.0 | 7.2 | 6.0 | 7.2 |
| $IC_{50}$ | 4.0 | 3.8 | 3.5 | 3.2 | <2.0 | <2.0 | <2.0 | <2.0 |

As to the $IC_{50}$ values for the MKN-28 cell [when the PBS (pH 6.0) was used], the Agg-IgG coated liposome formulation containing the MKN-28 DNase [i.e., "LP-DN"] was 24.5, the Agg-IgG coated liposome (without the MKN-28 DNase) [i.e., "LP(-DN)"] was 4.3, the MKN-28 DNase (without the Agg-IgG coated liposome) [i.e., "DN(-LP)"] was 15.0, and only the PBS buffer [i.e., "PBS"] was 2.7.

As to the $IC_{50}$ values for the MKN-28 cell [when the PBS (pH 7.2) was used], the Agg-IgG coated liposome formulation containing the MKN-28 DNase was 26.0, the Agg-IgG coated liposome (without the MKN-28 DNase) was 3.5, the MKN-28 DNase (without the Agg-IgG coated liposome) was 15.0, and only the PBS buffer was 2.5.

As to the $IC_{50}$ values for the HEF cell [when the PBS (pH 6.0) was used], the Agg-IgG coated liposome formulation containing the MKN-28 DNase was 4.0, the Agg-IgG coated liposome (without the MKN-28 DNase) was 3.5, the MKN-28 DNase (without the Agg-IgG coated liposome) was <2.0, and only the PBS buffer was <2.0.

As to the $IC_{50}$ values for the HEF cell [when the PBS (pH 7.2) was used], the Agg-IgG coated liposome formulation containing the MKN-28 DNase was 3.8, the Agg-IgG coated liposome (without the MKN-28 DNase) was 3.2, the MKN-28 DNase (without the Agg-IgG coated liposome) was <2.0, and only the PBS buffer was <2.0.

When the PBSs were used as the reaction buffer, the $IC_{50}$ values for the MKN-28 cell were 2.7 (pH 6.0) and 2.5 (pH 7.2), and the $IC_{50}$ values for the HEF cell were <2.0 (pH 6.0) and <2.0 (pH 7.2). The results show that PBSs have no cytotoxicity and are excellent for the reaction buffer, and that the difference between pH 6.0 and 7.2 does not affect the $IC_{50}$ values.

When the liposome (without the DNase) was used, the $IC_{50}$ values for the MKN-28 cell were 4.3 (pH 6.0) and 3.5 (pH 7.2), and the $IC_{50}$ values for the HEF cell were 3.5 (pH 6.0) and 3.2 (pH 7.2). The concentration of liposome was 0.5 $\mu$mol/L or less. The results show that the liposome has no cytotoxicity at a concentration of not more than 0.5 $\mu$mol/L.

When the MKN-28 DNase (without the liposome) was used, the $IC_{50}$ values for the MKN-28 cell were 15.0 (pH 6.0) and 15.0 (pH 7.2), and the $IC_{50}$ values for the HEF cell were <2.0 (pH 6.0) and <2.0 (pH 7.2). The DNase alone exhibited the activity for inhibiting the proliferation of the MKN-28 cell, but did not exhibit the activity for inhibiting the proliferation of the HEF cell as a normal cell.

When the liposome-DNase was used, the $IC_{50}$ values for the MKN-28 cell were 24.5 (pH 6.0) and 26.0 (pH 7.2), and the $IC_{50}$ values for the HEF cell were 4.0 (pH 6.0) and 3.8 (pH 7.2). The liposome-DNase exhibited a high $IC_{50}$ value for the MKN-28 cell, but had little cytotoxicity for the HEF cell as a normal cell.

The $IC_{50}$ value of the liposome-DNase was superior to that of the DNase (without the liposome). The result shows that the increased activity can be obtained by embedding the DNase in the liposome.

As described above, the centrifugal supernatant as an active ingredient in the present invention may be applied to the use of an antitumor agent. Furthermore, a mixture thereof with the liposome is more effective as the active ingredient of the antitumor agent.

Example 8: Relationship between cell division and cell proliferation inhibitory activity

When a DNase acts on the DNA of a cancer cell, the DNase must be brought into direct contact with the DNA. As to a DNase located in cytoplasm, a nuclear membrane inhibits the contact. The nuclear membrane disappears only during the mitotic period (M phase). To examine the increased activity of the DNase for inhibiting a cell proliferation, various cultured cells in the M phase were prepared, and the inhibitory activity of the DNase-containing liposome with respect to the M-phase cells was examined.

A synthetic colchicine, colcemid (J. Radiat. Res., 14, 258-270, 1971) was used to prepare the M-phase cell. The optimum conditions for obtaining the living M-phase cell, and the conditions for evaluation the activity of inhibiting a cell proliferation by the MTT method were examined.

More particularly, cells were cultivated in a 25-cm$^2$ culture flask (Falcon, 3014, 50 mL) for 3 days. After the mono-layered cells were washed, a growth medium supplemented with 0.025 $\mu$g/mL of colcemid (Nakarai, 09356-74) was added, and incubated at 37°C for 6 hours. After the cells were washed gently, the culture flask was gently shaken to collect cells removed from the bottom of the flask. The collected cells were washed with the medium (GM) by centrifugation to remove colcemid. The washed cells were suspended in the growth medium and incubated.

The incubated cells were observed under a microscope at intervals of an hour; an appearance of mitotic cells is shown in Table 19. The appearance of mitotic cells was judged by an appearance of round cells as an index.

Although almost cultured cells adhere to the glass surface and are thinly spread, mitotic cells become round and tend to leave the glass surface. Therefore, an appearance of round cells which tend to leave the monolayer was used as the index.

In Table 19, "-" means that the round cells appeared at a percentage of 0% with respect to the whole cells, "±" means that the round cells appeared at a percentage less than 5%, and "+" means that the round cells appeared at a percentage from 5% to less than 30%. The numbers in parentheses of "Round cells/flask" are numbers of positive cells strained by trypan blue.

Table 19

| | Appearance of round cells | | | | | Round cells/flask |
|---|---|---|---|---|---|---|
| Cell | 1hr | 2hr | 3hr | 4hr | 5hr | |
| MKN-28 | - | ± | + | + | + | 93000 (0) |
| MRC-5 | - | ± | + | + | + | 153000 (0) |

The yields of colcemid-treated cells (number of cells after the treatment/number of cells before the treatment) were 1.4% (93,000/6,300,000) for the MKN-28 cell and 29% (153,000/523,000) for the MRC-5 cell.

Next, the cells obtained by the treatment with 0.025 μg/mL of colcemid at 37°C for 6 hours were dispensed into each well in a 96-well plate to examine a growth activity of each mitotic cell. More particularly, when the mitotic MKN-28 cells (9300, 4650, 2325, and 1162 cells/well) were dispensed into each well and incubated at 37°C for 5 days, it was found that the cell density of 9300 cells/well or more was preferable to the MTT assay. In this connection, the growth activity was slightly lowered. When the mitotic MRC-5 cells (15300, 7650, 3825, and 1912 cells/well) were dispensed into each cell, no monolayer specific for fibroblasts was formed (cells grew, but confusion was observed), but it is considered that the cell density of 7650 or 3825 cells/well may be used in the MTT assay.

From the above results, although the conventional MTT assay was generally carried out under the conditions in which cells were used at the cell density of 5000 cells/well and the cultivation was carried out at 37°C for 4 days, it is considered that the MTT assay of the mitotic cells obtained by the colcemid treatment may be preferably carried out at the cell density of 10000 cells/well (MKN-28 cell) or 8000 cells/well (MRC-5 cells). In the MKN-28 cell, the growth activity was lowered in comparison with the normal cultured cell (i.e., not treated with colcemid), and dead cells were observed after 3 to 4 days from the beginning of the cultivation. In the MRC-5 cell, not only was the growth activity lowered, but also the activity of a cell proliferation was confused, and did not form the typical monolayer. The following MTT assay was carried out under the above conditions.

Example 9: Evaluation of liposome formulation containing purified DNase derived from MKN-28 cell: activity of inhibiting proliferation of various cells

In this example, the purified DNase derived from the MKN-28 cell [that is, the purified DNase obtained by purifying the centrifugal supernatant obtained in Example 1 through the Sephacryl S-300 HR in accordance with the procedure described in Example 2, and further purifying the resulting fraction by the ion-exchange chromatography in accordance with the procedure described in Example 3] was used for preparing a liposome formulation, and the activity thereof for inhibiting the proliferation of the MKN-28 cell was examined.
More particularly, as the purified DNase derived from the MKN-28 cell, the peak fractions obtained in Example 3 having the DNase activity, i.e., fraction No. 5 [50 mmol/L Tris-HCl (pH 7.5) + 0.08 mol/L NaCl] and fraction No.6 [50 mmol/L Tris-HCl (pH 7.5) + 0.10 mol/L NaCl] were used. The liposome was prepared in accordance with the procedure described in Example 7, except that the purified DNase obtained in Example 3 was used instead of the centrifugal supernatant obtained in Example 1, and that 50 mmol/L Tris-HCl (pH 7.5) supplemented with 0.08 mol/L NaCl was used as the reaction buffer, instead of the PBS. The MTT method was carried out in accordance with the procedure described in Example 7.

An amount of the purified MKN-28 DNase contained in the resulting Agg-IgG coated liposome formulation (suspension) containing the purified MKN-28 DNase was 17 units/100 μL. The "1 unit" as used herein means an amount of DNase capable of completely digesting 1 μg of λDNA at 37°C for an hour. The amount of DNase contained in the liposome formulation was determined by centrifuging 100 μL of the liposome formulation to thereby remove the supernatant, adding 100 μL of the PBS solution containing 0.2% Triton X-100 to thereby dissolve the liposome, and evaluating the activity of the λDNA digestion. It was previously confirmed that Triton X-100 did not affect the DNase activity.

In this example, the activity of the purified MKN-28 DNase for inhibiting the proliferation of the MKN-28 cell or the MRC-5 cell was evaluated by a cell-suspension method and a monolayer method.

In the cell-suspension method, double-diluted series (undiluted, 1/2, 1/4, 1/8, 1/16, and 1/32; 50 μL/well) of the Agg-IgG coated liposome formulation containing the purified MKN-28 DNase prepared with the GM were dispensed into each well. Further, 50 μL of a suspension of the MKN-28 cell or the MRC-5 cell (5000 cells/50 μL) was added, and incubated at 37°C in a $CO_2$ incubator. After 4 days from the beginning of the incubation, each $IC_{50}$ value was calculated by the MTT method.
As a control, double-diluted series of Agg-IgG coated liposome (without the purified MKN-28 DNase), double-diluted series of purified MKN-28 DNase (without the Agg-IgG coated liposome), and the reaction buffer were used, instead of the double-diluted series of Agg-IgG coated liposome formulation containing the purified MKN-28 DNase.

In the monolayer method, aliquots of a suspension of the MKN-28 cell or the MRC-5 cell (5000 cells/100 μL) were dispensed into each well of a 96-well microplate (100 μL/well), and cultured at 37°C for 20 hours. After the culture medium was removed from each well, double-diluted series (1/2, 1/4, 1/8, 1/16, and 1/32; 100 μL/well) of the Agg-IgG coated liposome formulation containing the purified MKN-28 DNase prepared with the GM were dispensed into each well, and cultured at 37°C. After 4 days from the beginning of the incubation, each $IC_{50}$ value was calculated by the MTT method.
As a control, double-diluted series of Agg-IgG coated liposome (without the purified MKN-28 DNase), double-diluted series of purified MKN-28 DNase (without the Agg-IgG coated liposome), and the reaction buffer (0.5 mmol/L Tris-HCl (pH 7.5) supplemented with 0.8 mol/L NaCl) were used, instead of the double-diluted series of Agg-IgG coated liposome formulation containing the purified MKN-28 DNase.
The result is shown in Table 20.
Table 20

| Cell | Method | LP-DN | LP(-DN) | DN(-LP) | PBS |
|---|---|---|---|---|---|
| MKN-28 | Cell-suspension | 5.3 | 2.7 | 2.7 | 2.8 |
| | Monolayer | <2.0 | 2.6 | 2.0 | 2.4 |
| MRC-5 | Cell-suspension | <2.0 | <2.0 | <2.0 | <2.0 |
| | Monolayer | <2.0 | <2.0 | <2.0 | <2.0 |

As shown in Table 20, when the MKN-28 cell was evaluated by the cell-suspension method, the $IC_{50}$ value of the Agg-IgG coated liposome formulation containing the purified MKN-28 DNase [i.e., "LP-DN"] was 5.3, that of the Agg-IgG coated liposome (without the purified MKN-28 DNase) [i.e., "LP(-DN)"] was 2.7, that of the purified MKN-28 DNase (without the Agg-IgG coated liposome) [i.e., "DN(-LP)"] was 2.7, and that of only the reaction buffer was 2.8. From the result, the Agg-IgG coated liposome formulation containing the purified MKN-28 DNase apparently exhibited the activity of inhibiting the proliferation of the MKN-28 cell. In contrast, the inhibitory activity was not observed in the monolayer method. Furthermore, when the MRC-5 cell as a normal cell was used, each $IC_{50}$ value was <2.0 by the cell-suspension method and the monolayer method, and the inhibitory activity was not observed.
The concentration of liposome was 0.5 μmol/L or less. As previously mentioned, liposome has no cytotoxicity at a concentration of 0.5 μmol/L or less, and it is considered that the result is not involved in the liposome concentration.

Example 10: Activity of Agg-IgG coated liposome formulation containing purified DNase derived from MKN-28 cell for inhibiting proliferation of M-phase cell

To enhance the activity of inhibiting a cell proliferation, cells to be treated were adjusted to the mitotic period in which the nuclear membrane disappeared (i.e., M phase), so that the DNase might be brought into contact with the DNA to be more easily digested. That is, cells were treated with a synthetic colchicine, colcemid, as described in Example 8.
More particularly, the MKN-28 cells or the MRC-5 cells were cultivated for 3 days, and each culture medium was changed to the growth medium supplemented with 0.025 μg/mL of colcemid. After the incubation at 37°C for 6 hours, the culture flask was gently shaken to collect cells removed from the bottom of the flask. The collected cells were washed with the normal growth medium without colcemid. Double-diluted series (undiluted, 1/2, 1/4, 1/8, 1/16, and 1/32; 50 μL/well) of the Agg-IgG coated liposome formulation containing the purified MKN-28 DNase prepared with the GM were dispensed into each well of a 96-well microplate.
Next, 50 μL of a suspension of the MKN-28 (M phase) cell (10000 cells/50 μL) or the MRC-5 (M phase) cell (8000 cells/50 μL) was added, and incubated at 37°C in a $CO_2$ incubator. After 4 days from the beginning of the incubation, each $IC_{50}$ value was calculated by the MTT method.
As a control, double-diluted series of Agg-IgG coated liposome (without the purified MKN-28 DNase), double-diluted series of purified MKN-28 DNase (without the Agg-IgG coated liposome), and the reaction buffer were used, instead of the double-diluted series of Agg-IgG coated liposome formulation containing the purified MKN-28 DNase.
The result is shown in Table 21.
As shown in Table 21, when the MKN-28 cell treated with colcemid was used, the $IC_{50}$ value of the Agg-IgG coated liposome formulation containing the purified MKN-28 DNase [LP-DN] was 9.3, and the activity of inhibiting the proliferation of the MKN-28 cell treated with colcemid was detected. In contrast, when the MRC-5 cell as a normal cell was used, each $IC_{50}$ value was <2.0, and the inhibitory activity was not observed.
As described above, the DNase of the present invention may be applied to the use of an antitumor agent. Furthermore, a mixture thereof with the liposome is more effective as the active ingredient of the antitumor agent. Additionally, the use of the DNase (or the mixture thereof) together with colcemid is also effective.

Table 21

| Cell | Method | LP-DN | LP(-DN) | DN(-LP) | PBS |
|------|--------|-------|---------|---------|-----|
| MKN-28 | Cell-suspension | 9.3 | 5.0 | 4.5 | 2.8 |
| MRC-5 | Cell-suspension | <2.0 | <2.0 | <2.0 | <2.0 |

Example 11: Evaluation of Agg-IgG coated liposome formulation containing restriction enzyme XspI : activity of inhibiting cell proliferation

In this example, a restriction enzyme XspI (Takara, 1095A) was used instead of the MKN-28 DNase to prepare a liposome formulation containing the restriction enzyme XspI, in accordance with the procedures described in Example 7, and the activity of inhibiting a cell proliferation was evaluated by the MTT method (the cell-suspension method or the monolayer method). As cells for the evaluation, human lung cancer cell line A549 (RCB0098, Riken or ATCC CCL185, ATCC), human stomach cancer cell line MKN-28, and human fetal lung fibroblast MRC-5 (as a normal cell) were used. In protooncoges N-ras, Ha-ras, and Ki-ras, the codons at the 11th and 12th are GCT (Ala) and GGT (Gly), and the corresponding codons in the A549 cell are changed to GCT (Ala) and AGT (Ser). The nucleotide sequence "CTAG" in the sequence GCTAGT in the A549 cell is a recognition sequence "C:TAG" (":" means a cleavage site) of the restriction enzyme XspI. A mutation which causes a transformation is not identified in the MKN-28 cell.

The results ($IC_{50}$ values) are shown in Table 22 and Table 23. In Table 22, $IC_{50}$ values are indicated as a dilution of liquid. In Table 23, $IC_{50}$ values are indicated as a unit of "XspI units/well/100 $\mu$L". In Tables 22 and 23, "LP-Xsp", "LP(-Xsp) ", "Xsp(-LP)", and "RB" mean the Agg-IgG coated liposome formulation containing the restriction enzyme XspI, the Agg-IgG coated liposome (without the restriction enzyme XspI), the restriction enzyme XspI (without the Agg-IgG coated liposome), and only the reaction buffer [20 mmol/L Tris-HCl (pH 8.5), 10 mmol/L $MgCl_2$, 1 mmol/L DTT, and 100 mmol/L KCl], respectively.

Table 22

| Cell | Method | $IC_{50}$ value (dilution) | | | |
|------|--------|--------|---------|----------|-----|
| | | LP-Xsp | LP(-Xsp) | Xsp(-LP) | RB |
| A549 | Cell-suspension | 12.0 | 6.2 | 11.5 | 5.0 |
| | Monolayer | 4.7 | 3.5 | 4.3 | 3.4 |
| MKN-28 | Cell-suspension | 7.3 | <2.0 | 7.2 | <2.0 |
| | Monolayer | 15.0 | 2.9 | 19.0 | 5.2 |
| MRC-5 | Cell-suspension | 3.0 | <2.0 | 2.2 | 2.0 |
| | Monolayer | 2.6 | <2.0 | 2.4 | 2.4 |

Table 23

| Cell | Method | $IC_{50}$ value (XspI units/well/100 $\mu$L) | | | |
|------|--------|--------|---------|----------|-----|
| | | LP-Xsp | LP(-Xsp) | Xsp(-LP) | RB |
| A549 | Cell-suspension | 0.68 | 1.35 | 0.68 | 1.6 |
| | Monolayer | 1.7 | 2.3 | 1.9 | 2.3 |
| MKN-28 | Cell-suspension | 1.25 | >4.0 | 1.27 | >4.0 |
| | Monolayer | 0.6 | 2.6 | 0.4 | 1.5 |
| MRC-5 | Cell-suspension | 2.95 | >4.0 | 3.5 | 4.0 |
| | Monolayer | 3.0 | >4.0 | 3.2 | 3.2 |

As shown in Table 22, when the human lung cancer cell line A549 was evaluated by the cell-suspension method, the $IC_{50}$ value of the Agg-IgG coated liposome formulation containing the restriction enzyme XspI was 12.0, and that of only the restriction enzyme XspI was 11.5. Both exhibited the activity of inhibiting the proliferation of the A549 cell. When the human stomach cancer cell line MKN-28 was evaluated by the cell-suspension method, the $IC_{50}$ value of the Agg-IgG coated liposome formulation containing the restriction enzyme XspI was 7.3, and that of only the restriction enzyme XspI was 7.2. When the same MKN-28 cell was evaluated by the monolayer method, the $IC_{50}$ value of the Agg-IgG coated liposome formulation containing the restriction enzyme XspI was 15.0, and that of only the restriction enzyme XspI was 19.0. All cases exhibited the activity of inhibiting the proliferation of the MKN-28 cell.

In contrast, when the MRC-5 cell as a normal cell was used, the Agg-IgG coated liposome formulation containing the restriction enzyme XspI and the restriction enzyme XspI alone did not exhibit the activity of inhibiting the proliferation of the MRC-5 cell. From the result, it was confirmed that the antitumor agent of the present invention does not act on normal cells.

Example 12: Activity of Agg-IgG coated liposome formulation containing restriction enzyme XspI for inhibiting proliferation of of M-phase cell

In this example, the activity of the Agg-IgG coated liposome formulation containing the restriction enzyme XspI for inhibiting a proliferation of each M-phase cell was evaluated in accordance with the methods described in Example 10. The results are shown in Table 24 and Table 25.

As shown in Tables 24 and 25, the antitumor agent of the present invention exhibited the activity of inhibiting a proliferation of human lung cancer cell line A549 or human stomach cancer cell line MKN-28, but did not act on normal cells.

Table 24

| | | $IC_{50}$ value (dilution) | | | |
|---|---|---|---|---|---|
| Cell | Method | LP-Xsp | LP(-Xsp) | Xsp(-LP) | RB |
| A549 | Cell-suspension | 8.3 | 3.7 | 6.0 | 3.4 |
| MKN-28 | Cell-suspension | 12.0 | 4.6 | 7.9 | 3.0 |
| MRC-5 | Cell-suspension | 2.5 | <2.0 | 2.7 | 2.0 |

Table 25

| | | $IC_{50}$ value (XspI units/well/100 $\mu$L) | | | |
|---|---|---|---|---|---|
| Cell | Method | LP-Xsp | LP(-Xsp) | Xsp(-LP) | RB |
| A549 | Cell-suspension | 0.94 | 2.3 | 1.3 | 2.2 |
| MKN-28 | Cell-suspension | 0.65 | 1.65 | 1.2 | 2.6 |
| MRC-5 | Cell-suspension | 3.0 | >4.0 | 2.9 | 4.0 |

INDUSTRIAL APPLICABILITY

As above, the present invention was explained with reference to particular embodiments, but modifications and improvements obvious to those skilled in the art are included in the scope of the present invention.

**Claims**

1.   An antitumor agent comprising as an active ingredient a DNase.

2.   The antitumor agent according to claim 1, comprising as an active ingredient a complex of the DNase and a liposome.

3.   A DNase having the following properties:

(a) activity and substrate specificity: exhibiting an endonuclease activity;
(b) molecular weight: 48 to 43 kDa (determined by a gel filtration chromatography);
(c) optimum pH: pH 3.0 to 4.5;

(d) thermostability: the endonuclease activity is not inactivated by heating at 100°C for 10 minutes; and
(e) susceptibility to proteinase K treatment: the endonuclease activity is inactivated by a treatment with proteinase K at 37°C for 15 minutes.

4. A DNase having the following properties:

(a) activity and substrate specificity: exhibiting an endonuclease activity;
(b) molecular weight: 63 kDa (determined by a gel filtration chromatography);
(c) optimum pH: pH 3.0 to 4.5;
(d) thermostability: the endonuclease activity is inactivated by heating at 100°C for 10 minutes; and
(e) susceptibility to proteinase K treatment: the endonuclease activity is not inactivated by a treatment with proteinase K at 37°C for 15 minutes.

5. A pharmaceutical composition comprising the DNase according to claim 3 or 4 and a pharmaceutically or veterinarily acceptable ordinary carrier or diluent.

6. The antitumor agent according to claim 1 or 2, wherein the DNase is a restriction enzyme or the DNase according to claim 3 or 4.

7. Use of a DNase in the manufacture of an antitumor agent.

8. The use according to claim 7, wherein the DNase is a complex of the DNase and a liposome.

9. The use according to claim 7 or 8, wherein the DNase is a restriction enzyme or the DNase according to claim 3 or 4.

10. A method for treating or preventing cancer, comprising administering to a subject in need thereof a DNase in an amount effective in treating or preventing cancer.

11. The method according to claim 10, wherein the DNase is a complex of the DNase and a liposome.

12. The method according to claim 10 or 11, wherein the DNase is a restriction enzyme or the DNase according to claim 3 or 4.

## INTERNATIONAL SEARCH REPORT

International application No.

PCT/JP2006/307338

| A. CLASSIFICATION OF SUBJECT MATTER |
|---|
| *A61K38/46*(2006.01), *A61K9/127*(2006.01), *A61P35/00*(2006.01), *C12N9/16* (2006.01) |

According to International Patent Classification (IPC) or to both national classification and IPC

| B. FIELDS SEARCHED |
|---|
| Minimum documentation searched (classification system followed by classification symbols) |
| A61K38/46, A61K9/127, A61P35/00, C12N9/16 |

Documentation searched other than minimum documentation to the extent that such documents are included in the fields searched

| Jitsuyo Shinan Koho | 1922-1996 | Jitsuyo Shinan Toroku Koho | 1996-2006 |
|---|---|---|---|
| Kokai Jitsuyo Shinan Koho | 1971-2006 | Toroku Jitsuyo Shinan Koho | 1994-2006 |

Electronic data base consulted during the international search (name of data base and, where practicable, search terms used)
WPI, BIOSIS(STN), CAplus(STN), EMBASE(STN), MEDLINE(STN), JCHEM(JDream2), JMEDPlus(JDream2), IGAKU·YAKUGAKU YOKOSHU ZENBUN DATABASE

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | WO 96/41887 A1 (DEUTSCHES KREBSFORSCHUNGS-<br>ZENTRUM STIFTUNG DES ÖFFENTLICHEN RECHTS),<br>27 December, 1996 (27.12.96),<br>Abstract; Page 5, lines 4 to 20<br>& DE 19521046 C1    & EP 842278 A1<br>& JP 11-507825 A    & EP 842278 B1<br>& DE 59611293 G | 1,6,7,9<br>2,5,8 |
| X<br>Y | EP 754751 A1 (TANUMA, Seiichi),<br>22 January, 1997 (22.01.97),<br>Abstract; Claims; Page 23, lines 4 to 12<br>& WO 96/07735 A1    & JP 08-187079 A<br>& US 5821103 A    & EP 1045028 A1<br>& US 6143875 A    & DE 69522799 E<br>& JP 3378706 B2 | 1,6,7,9<br>2,5,8 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    24 April, 2006 (24.04.06) | Date of mailing of the international search report<br>    02 May, 2006 (02.05.06) |
|---|---|
| Name and mailing address of the ISA/<br>    Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2006/307338 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | WO 2004/032962 A1 (IMMUNOMEDICS, INC.), 22 April, 2004 (22.04.04), Abstract; Claims & AU 2002332087 A1      & EP 1558284 A1 | 1,6,7,9 |
| X | WO 2003/106497 A1 (IMMUNOMEDICS, INC.), 24 December, 2003 (24.12.03), Abstract; Claims & US 2004/057902 A1      & AU 2003250367 A1 & EP 1521775 A1             & KR 2005010055 A | 1,6,7,9 |
| X | WO 2003/068821 A2 (IMMUNOMEDICS, INC.), 21 August, 2003 (21.08.03), Abstract; Claims & US 2003/219433 A1      & AU 2003208415 A1 & KR 2004086383 A          & EP 1519959 A2 & JP 2006-500904 A         & CN 1662557 A | 1,6,7,9 |
| X | WO 2003/033654 A2 (IMMUNOMEDICS, INC.), 24 April, 2003 (24.04.03), Abstract; Claims & US 2003/148409 A1      & EP 1448780 A2 & AU 2002348437 A1         & JP 2005-507659 A & BR 200213303 A           & CN 1604966 A | 1,6,7,9 |
| X | WO 2001/34176 A1 (IMMUNOLYTICS INC.), 17 May, 2001 (17.05.01), Abstract; Claims & AU 200126179 A           & US 2002/061300 A1 & US 6428785 B1            & US 2002/106359 A1 & EP 1242110 A1            & CN 1384754 A & JP 2003-513931 A         & US 6913744 B2 | 1,6,7,9 |
| X | WO 2001/74905 A1 (ANTISOMA RESEARCH LTD.), 11 October, 2001 (11.10.01), Abstract; Claims & AU 200144307 A           & US 2002/122798 A1 | 1,6,7,9 |
| X Y | QUARLESS, Shelley A., Identification of a deoxyribonuclease activity in the nuclei of the cervical carcinoma HeLa, Cancer Biochemistry Biophysics, 1986, Vol.8, No.3, pp.211-20, Abstract | 4 3,5 |
| Y | NUZZO, F. et al., Chromosomal aberrations induced in human cultured cells by liposome-encapsulated deoxyribonuclease I, Mutation Research, 1987, Vol.177, No.1, pp.117-24, Abstract | 2,8 |

Form PCT/ISA/210 (continuation of second sheet) (April 2005)

**INTERNATIONAL SEARCH REPORT**

International application No.

PCT/JP2006/307338

C (Continuation). DOCUMENTS CONSIDERED TO BE RELEVANT

| Category* | Citation of document, with indication, where appropriate, of the relevant passages | Relevant to claim No. |
|---|---|---|
| Y | ZAJAC-KAYE, Maria et al., DNAase I encapsulated in liposomes can induce neoplastic transformation of Syrian hamster embryo cells in culture, Cell, 1984, Vol.39, No.3, Pt.2, pp.427-37, Abstract | 2,8 |
| Y | KATOH, Masaru et al., Alternative Splicing of the WNT-2B/WNT-13 Gene, Biochemical and Biophysical Research Communications, 2000, Vol.275, No.1, pages 209 to 216, Abstract | 3,5 |

Form PCT/ISA/210 (continuation of second sheet) (April 2005)

**INTERNATIONAL SEARCH REPORT**

International application No.

PCT/JP2006/307338

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 10-12
   because they relate to subject matter not required to be searched by this Authority, namely:
   Claims 10 to 12 pertain to [methods for treatment of the human body by therapy] and thus relate to a subject matter which this International Searching Authority is not required, under the provisions of the PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

The "special technical feature" of claims 1, 2, 6-12 relates to a "use of DNase as an antitumor agent". The "special technical feature" of claim 3 and part of claim 5 relates to a "use of beyond the DNase per se having a character of claim 3 and an antitumor agent thereof as a pharmaceutical" which is only a small portion of the above-mentioned DNase. The "special technical feature" of claim 4 and part of claim 5 relates to a "use of beyond the DNase per se having a character of claim 4 and an antitumor agent thereof as a pharmaceutical" which is only a small portion of the above-mentioned DNase and different from that of claim 3. There is no technical relationship among these three inventions involving one or (continued extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

Form PCT/ISA/210 (continuation of first sheet (2)) (April 2005)

**INTERNATIONAL SEARCH REPORT**

| International application No. |
|---|
| PCT/JP2006/307338 |

Continuation of Box No. III of continuation of first sheet (2)

more of the same or corresponding special technical features, therefore these inventions are not so linked as to form a single general inventive concept.

Form PCT/ISA/210 (extra sheet) (April 2005)

**EP 1 870 108 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *British Journal of Cancer,* 1972, vol. 26, 239
- *J. Virol. Methods,* 1988, vol. 20, 309-321
- *Journal of Virological Methods,* 1988, vol. 20, 309
- *Biochemistry,* 1976, vol. 15, 452
- *Biochemistry,* 1976, vol. 15 (2), 452-460
- *J. Infect. Dis,* 1991, vol. 163, 270-275
- *J. Radiat. Res,* 1971, vol. 14, 258-270